# EUROPEAN PATENT SPECIFICATION

(11) **EP 2 877 183 B1**
(45) Date of publication and mention of the grant of the patent: **16.12.2020**
(21) Application number: 13822094.2
(22) Date of filing: 19.07.2013
(51) Int. Cl.: A61K 31/5685, A61P 15/00, A61P 15/02, A61P 15/12

(54) **IMPROVEMENT OF SEXUAL AROUSAL, SEXUAL DESIRE, ORGASM AND/OR PLEASURE FOLLOWING INTRAVAGINAL PRASTERONE (DHEA) ADMINISTRATION IN WOMEN NOT SUFFERING OR INDEPENDENTLY FROM DYSPAREUNIA OR OTHER SYMPTOMS OF VULVO-VAGINAL ATROPHY**
VERBESSERUNG VON SEXUELLER ERREGUNG, SEXUELLEM VERLANGEN, ORGASMUS UND/ODER VERGNÜGEN INTRAVAGINALE NACH INTRAVAGINALER PRASTERON (DHEA)-VERABREICHUNG BEI FRAUEN, DIE NICHT VON DYSPAREUNIA ODER ANDEREN SYMPTOMEN VON VULVOVAGINALER ATROPHIE BETROFFEN SIND ODER UNABHÄNGIG DAVON
AMÉLIORATION DE L'EXCITATION SEXUELLE, DU DÉSIR SEXUEL, DE L'ORGASME ET/OU DU PLAISIR À LA SUITE D'UNE ADMINISTRATION DE PRASTÉRONE (DHA) INTRA VAGINALE CHEZ DES FEMMES NE SOUFFRANT PAS DE DYSPAREUNIE OU D'AUTRES SYMPTÔMES D'ATROPHIE VULVO-VAGINALE OU INDÉPENDAMMENT DE CEUX-CI

(30) Priority: 25.07.2012 US 201261675717 P; 16.07.2013 US 201313942977
(43) Date of publication of application: 03.06.2015
(73) Proprietor: Endorecherche, Inc., Québec City, Québec G1W 2J5 (CA)
(72) Inventor: LABRIE, Fernand, Québec City, Québec G1W 2J5 (CA)
(74) Representative: Carpintero Lopez, Francisco
(86) International application number: PCT/CA2013/000654
(87) International publication number: WO 2014/015416

(56) References cited:
- WO-A1-2006/042409
- WO-A1-2009/021323
- WO-A1-2013/012430
- LABRIE, FERNAND ET AL.: '`Effect of intravaginal dehydroepiandrosterone (Prasterone) on libido and sexual dysfunction in postmenopausal women' MENOPAUSE: THE JOURNAL OF THE NORTH AMERICAN MENOPAUSE SOCIETY vol. 16, no. 5, pages 923 - 931, XP055182004
- PANJARI, M ET AL.: 'DHEA therapy for women: effect on sexual function and wellbeing' HUMAN REPRODUCTION UPDATE vol. 13, no. 3, 2007, pages 239 - 248, XP008084981
- MESTON, CINDY: 'The Psychophysical Assessment of Female Sexual Function' JOURNAL OF SEX EDUCATION AND THERAPY vol. 25, no. 1, pages 6 - 16, XP028744028
- LABRIE ET AL: "Intravaganal dehydroepiandrosterone a physiological and highhly efficient treatment of vaginal atrophy", MENOPAUSE, vol. 16, no. 5, 3009, - 2009, pages 907-922,
- LABRIE ET AL: "Lack of influence of dyspareunia on the beneficial effect of intravaginal Prasterone on sexual dysfunction in postmenapausal women.", J. SEX. MED, vol. 11, 2014, - 2014, pages 1766-1785,

## Description

### FIELD OF THE INVENTION

The present invention relates to the intravaginal use of prasterone (DHEA, dehydroepiandrosterone) for treating the following domains of female sexual dysfunction (FSD): female sexual arousal disorder (FSAD), female hypoactive sexual desire disorder (HSDD) and female orgasmic disorder (FOD) alone or in combination (including sexual interest arousal disorder (SIAD)...) in menopausal women not suffering or independently from dyspareunia (pain at sexual activity) or other symptoms of vulvo-vaginal atrophy.

### BACKGROUND

It is known from WO 94/16709 that the sex steroid precursor DHEA can be utilized for the treatment and/or prevention of vulvovaginal atrophy or diminished libido. WO 06/42409 teaches inter alia novel methods for treating or reducing the likelihood of acquiring vaginal dysfunction, particularly of vaginal dryness and dyspareunia, frequently accompanied by sexual dysfunction and low sexual desire by intravaginal administration of sex steroid precursors like DHEA. WO 09/21323 discloses certain DHEA doses and compositions, particularly vaginal suppositories, for treating menopause symptoms like vaginal atrophy, dyspareunia and dryness as well as diminished libido.

Recent data have shown that the intra-vaginal administration of DHEA for twelve weeks shows beneficial effects on the four domains of sexual dysfunction recognized in the Diagnostic and Statistical Manual (DSM)-IV, namely sexual desire, sexual arousal, orgasm and dyspareunia (American Psychiatric Association 2000; Labrie, Archer et al. 2009b).

Since, at the doses used, DHEA acts exclusively in the vagina following its local conversion into androgens and/or estrogens by the mechanisms of intracrinology (Labrie 1991; Labrie, Luu-The et al. 2005) without systemic exposure or significant changes in serum testosterone or estradiol (Labrie, Cusan et al. 2008a; Labrie, Cusan et al. 2008b), the newly discovered effect of DHEA challenges the existing paradigm for the pathogenesis and treatment of FSD (Female Sexual Dysfunction). It has been postulated that excitatory and inhibitory factors in the brain control sexual functioning. Following this concept, the female sexual desire disorder would be due to an imbalance between these excitatory and inhibitory factors. Among other factors, the neurotransmitters dopamine and serotonin are important to sexual function. Dopamine appears to increase sexual desire, the subjective sensation of arousal and the desire to continue sexual activity once the stimulation has begun whereas serotonin would have an inhibitory effect on sexual desire. Other neurotransmitters involved are norepinephrine and oxytocin with excitatory effects, and prolactin and opioids with inhibitory effects

With this concept of the CNS playing a major role in female sexual functioning, the treatment of FSD has focused on drugs aiming at a direct effect on the brain, namely testosterone, flibanserin or other agents, with, however, only limited success.

The new and completely unexpected observation that a local intravaginal administration of DHEA without systemic exposure has a beneficial effect on symptoms of female sexual dysfunction suggests that there must be other non-humoral mechanisms responsible to influence sexual function at the level of the CNS. Sexual dysfunction is a common problem with rates of up to 50% self-reported among women in community studies using questionnaires (Béjin 1994; Kontula and Haavio-Mannila 1995; Burwell, Case et al. 2006). The prevalence of sexual dysfunction increases after ovariectomy and with age (Nathorst-Boos and von Schoultz 1992; Laumann, Paik et al. 1999) and a higher incidence is observed in postmenopausal compared to premenopausal women (Hallstrom 1977; Osborn, Hawton et al. 1988; Hallstrom and Samuelsson 1990; Dennerstein, Smith et al. 1994; Bancroft and Cawood 1996; Avis, Stellato et al. 2000; Dennerstein, Dudley et al. 2001), an effect most likely related, up to an unknown extent, to the general hormonal deficiency of menopause.

While psychological factors are believed to play an important role in the loss of sexual desire/interest and arousal, many studies have reported a beneficial effect of androgens on sexual function in women (Sherwin and Gelfand 1985; Sherwin and Gelfand 1987; Davis, McCloud et al. 1995; Sarrel, Dobay et al. 1998; Tuiten, Van Honk et al. 2000; Tuiten, van Honk et al. 2002; Goldstat, Briganti et al. 2003; Lobo, Rosen et al. 2003; Shifren, Davis et al. 2006; Hubayter and Simon 2008). These observations have resulted in an increased use of testosterone for this indication (Hulter and Lundberg 1994; Davis and Tran 2001), although some controversy still persists about the efficacy of androgens on sexual dysfunction (Myers, Dixen et al. 1990; Basson 2007).

While low DHEA activity has been indicated as cause of the symptoms of hormone deficiency in postmenopausal women (Labrie 2010a), recent studies have reported lower serum levels of DHEA and/or DHEA-S in women with sexual dysfunction compared to controls (Davis, Davison et al. 2005; Basson, Brotto et al. 2010). Since the formation of sex steroids from DHEA is tissue-specific, the use of DHEA offers the opportunity to provide physiological levels of androgens and/or estrogens which are made, act and are inactivated locally in each tissue by intracrine mechanisms and where only the inactive metabolites are released in the circulation (Labrie, Luu-The et al. 2005). The use of DHEA thus avoids the systemic effects of sex steroids which are necessarily delivered through the general circulation following oral or percutaneous administration (Labrie, Diamond et al. 1997; Labrie 2001; Lasco, Frisina et al. 2001; Labrie, Luu-The et al. 2003).

Since it is recognized that estrogens improve vaginal atrophy symptoms by an action in the most superficial layer of the vagina without affecting sexual dysfunction (Lobo, Rosen et al. 2003; Long, Liu et al. 2006; Raghunandan, Agrawal et al. 2010) while DHEA corrects both vaginal atrophy symptoms and sexual dysfunction (Labrie, Archer et al. 2009b; Labrie, Archer et al. 2009a), it appears logical to suggest that vaginal atrophy and sexual dysfunction (called WSD, vulvovaginal sexual dysfunction) are two separate medical entities both caused by hormone deficiency, in analogy with osteoporosis and hot flushes which are two different medical entities commonly caused by a lack of DHEA activity. While estrogens improve vaginal atrophy by an action on the superficial vaginal layer, androgens derived from local DHEA transformation improve sexual dysfunction by acting also in the vagina but at a different site and by different local mechanisms. In fact, it has recently been shown that DHEA administration increases the density of nerve fibers in the two deepest layers of the vagina in the rat, namely in the lamina propria and muscularis (Pelletier, Ouellet et al. 2012a; Pelletier, Ouellet et al. 2012b).

Since pain at sexual activity is a predominant symptom in postmenopausal women suffering from vaginal atrophy (Labrie, Archer et al. 2009a) while being considered as being one of the four domains of FSD (American Psychiatric Association 2000), we have analyzed the potential differences in the severity scores of sexual dysfunction parameters, namely desire, arousal and orgasm and the response of these severity scores to intravaginal DHEA administration in women with and without moderate or severe pain at sexual activity at baseline in our study ERC-210 (Labrie, Archer et al. 2009b). The finding of a similar effect of DHEA in women with vaginal atrophy suffering from moderate to severe (MS) pain (a well recognized domain of sexual dysfunction) and those not suffering from MS pain would indicate that vaginal atrophy (pain at sexual activity, vaginal dryness and irritation/itching) and sexual dysfunction caused by dysfunction of the vaginal nerve fibers located in the second (lamina propria) and third (muscularis) layers are two completely separate entities.

### SUMMARY OF THE INVENTION

The present invention relates to the intra-vaginal use of DHEA for treating sexual arousal, sexual desire and/or orgasm problems alone or in combination in women not suffering from dyspareunia or symptoms of vulvovaginal atrophy and also in premenopausal women having no symptoms/signs of vaginal atrophy.

Frequently, postmenopausal women suffer from reduced sexual arousal, reduced sexual desire, and/or difficulty with orgasm often associated with changes in the vulvar and vaginal tissues known as vulvovaginal atrophy (WA). However, reduced sexual desire, reduce arousal and difficulty with orgasm of so-far unknown etiology is also observed in postmenopausal women having eutrophic vaginal epithelium (= comparable to a premenopausal vagina without signs of atrophy).

The population of postmenopausal women can then be subdivided into three subpopulations: women with (1) moderate to severe VVA, (2) mild VVA, and (3) no VVA or normal vaginal epithelium. Particularly, Population (1) experiences vaginal dryness, which in turn can lead to moderate/severe pain during sexual activity, in turn potentially accompanied by reduced sexual desire and/or arousal and/or orgasm. To correct this bothersome condition, these patients were previously treated with estrogens, including DHEA, which is converted locally in the vagina to estradiol (E2) and testosterone (T), that corrects VVA and, as we have shown (Labrie, Archer et al. 2009b; Labrie, Archer et al. 2009a), also increases sexual desire, arousal and orgasm. The mechanisms leading to correction of VVA symptoms and of sexual dysfunction are, however, as shown in the present invention, different and involve different sites of action. In agreement with this suggestion, treatment with estrogens, while treating the symptoms of VVA does not correct sexual dysfunction.

Population (2) has less severe VVA symptoms, and is typically treated symptomatically with lubricants or moisturisers with limited success. Use of estrogens, lubricants and moisturisers in Populations (1) and (2) is known and, therefore, not inventive. However, during studies involving patients with and without moderate or severe pain at sexual activity, a surprise observation, which is the basis of the described invention was made:
Contrary to the expectation, vaginal treatment with DHEA increased to the same extent sexual desire, arousal and orgasm in women with and without moderate or severe pain at sexual activity. Postmenopausal women without the symptom of moderate to severe pain at sexual activity were treated with equal success in the presented study and responded unexpectedly equally well with an improvement of sexual desire, sexual arousal and ability to orgasm. Such data indicate that the hormone deficiency in postmenopausal women can cause vaginal atrophy symptoms, especially dyspareunia or pain at sexual activity and, independently, cause sexual dysfunction (low desire, low arousal and difficulty with orgasm). Our recent data dissociate the two diseases (sexual dysfunction and vulvo-vaginal atrophy) and indicate that intravaginal DHEA can be successfully used to treat sexual dysfunction independently from the severity or presence of vaginal atrophy.

### DETAILED DESCRIPTION OF THE INVENTION

The present data show that despite pain at sexual activity being one of the four domains of FSD with decreased sexual desire, decreased sexual arousal and persistent difficulty or inability in achieving orgasm, according to the Diagnostic and Statistical Manual (DSM) IV (American Psychiatric Association 2000), moderate to severe dyspareunia does not have a significant impact on the benefits observed on desire, arousal and orgasm following the local intravaginal action of DHEA.

Strong interrelationships between the various domains of FSD are well recognized. In a cross-sectional study performed in 1002 women aged 20 to 70 years using the Sexual Function Questionnaire (SFQ) and the Female Sexual Distress Scale (FSDS), low desire was more associated with relationship factors than age and menopause while low genital arousal and low orgasmic function were more associated with physiological and psychological factors (Haves, Dennerstein et al. 2008). Sexual distress, on the other hand, was associated with both psychological and relationship factors. Sexual distress was associated with higher depression scores and negative feelings for the partner. Moreover, sexual distress could be predicted by prior negative feelings for the partner and a greater decline in sexual function scores.

A higher correlation has been found between the FSFI (Female Sexual Function Index) arousal (psychological) and desire constructs than between the FSFI arousal and lubrication which are supposed to measure the same construct (Wiegel, Meston et al. 2005). This could suggest that sexual desire and arousal (psychological) could be members of the same construct and are too interrelated to be measured separately. In the sildenafil trials aimed at treating FSAD (Female Sexual Arousal Disorder), there was a more than 50% overlap with women also meeting the criteria for HSDD (Hypoactive Sexual Desire Disorder) and FOD (Female Orgasmic Disorder) (Caruso, Intelisano et al. 2001; Basson, Mclnnes et al. 2002; Berman, Berman et al. 2003; Caruso, Rugolo et al. 2006).

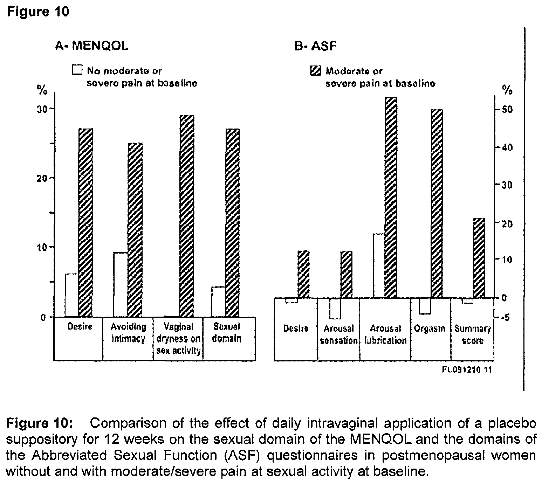

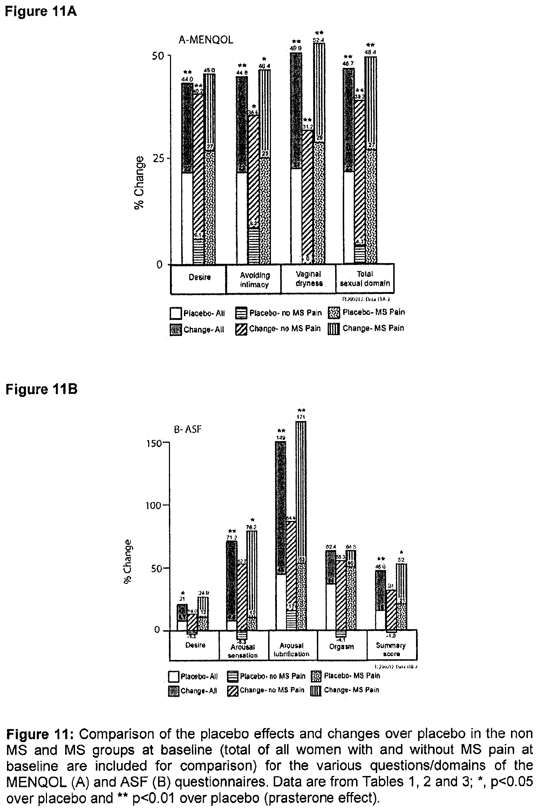

In the present analysis in a population of women showing or not showing moderate to severe pain at sexual activity at baseline, it is observed that the placebo effect of treatment with intravaginal DHEA is practically entirely attributable to the changes observed with placebo in the group of women having moderate to severe (MS) pain at sexual activity at baseline, while practically no placebo effect on sexual dysfunction was seen in women with no MS pain at baseline (Figures 10 and 11; Tables 1 and 2). As a corollary to this observation, women having MS pain had somewhat higher severity scores at baseline in both the MENQOL and ASF questionnaires (Tables 4 and 5).

It is pertinent, at this stage, to provide some information on the different mechanisms of action of intra-vaginal DHEA likely to be involved in sexual dysfunction on one hand and on vaginal atrophy, on the other hand. The important clinical health issues related to hormone deficiency and facing women at menopause pertain to hot flushes, vaginal atrophy, bone loss, loss of muscle mass and strength, fat accumulation, type 2 diabetes, sexual dysfunction, memory loss, cognition loss and possibly Alzheimer's disease (Labrie 2007). Due to the variable sensitivity of each tissue morphology and function to hormone deficiency, it now appears logical that hormonal deficiency in different postmenopausal women can lead to different levels of clinically detectable deficiency in any or all the above-mentioned symptoms: in some women, osteoporosis can be the predominant symptom or hormone deficiency consequence with no other apparent sign of menopause while, in other women, sexual dysfunction can be more predominant and in others, vaginal atrophy or skin atrophy, or muscle loss will be the most evident symptom or sign of hormone deficiency. It is of particular interest that many of the medical problems related to menopause have been found to respond positively to androgens and, in many cases, to the administration of DHEA when used at the proper dose and under appropriate experimental conditions (Davis, McCloud et al. 1995; Labrie, Diamond et al. 1997; Simon, Klaiber et al. 1999; Villareal and Holloszy 2004) (*see review:* (Labrie 2007; Labrie, Archer et al. 2009b; Labrie, Archer et al. 2009a; Labrie, Archer et al. 2009c; Labrie 2010b).

Concerning the androgenic aspect, it is important to consider that normal women produce an amount of androgens equivalent to approximately 50% of the androgens secreted in men (Labrie, Bélanger et al. 2006). However, since all androgens in women originate from DHEA (Labrie, Martel et al. 2011), the pool of androgens in women decreases markedly and progressively from the age of 30 years in parallel with the marked fail in the serum concentration of DHEA and DHEA-S. In fact, on average, women have already lost 60% of their DHEA and, consequently, androgens at time of menopause (Labrie, Luu-The et al. 2005; Labrie 2010a).

Most importantly, as mentioned above, after menopause, the only source of sex steroids is DHEA. Consequently, the presence or absence of symptoms related to hormonal deficiency can only be related to differences in DHEA availability between different women (Labrie 2010a; Labrie, Martel et al. 2011) as well as to differences of sensitivity to DHEA and also different metabolism in the different tissues of each woman. It thus appears logical to include androgenic replacement therapy at postmenopause. However, in order to maintain a physiological balance between androgens and estrogens in each cell and each tissue, only exogeneous DHEA permits the local formation of androgens and/or estrogens according to the physiology of each tissue. In addition to providing the appropriate levels of androgens and/or estrogens synthesized in each specific tissue by intracrine mechanisms, DHEA, an inactive molecule by itself, avoids systemic exposure to sex steroids, an issue raised by the WHI study and a long series of follow-up publications.

Sexual dysfunction is member of the spectrum of symptoms and medical problems mentioned above related to sex steroid deficiency in postmenopausal women. In fact several studies have reported diminished sexual desire or interest, decreased sexual receptivity and decreased sexual responsiveness in postmenopausal women (Hallstrom 1977; Osborn, Hawton et al. 1988; Hallstrom and Samuelsson 1990; Nathorst-Boos and von Schoultz 1992; Dennerstein, Smith et al. 1994; Bancroft and Cawood 1996; Laumann, Paik et al. 1999; Avis, Stellato et al. 2000; Dennerstein, Dudley et al. 2001). The well recognized data showing that estrogens correct vaginal atrophy symptoms without significantly affecting sexual dysfunction (Lobo, Rosen et al. 2003; Gonzalez, Viafara et al. 2004; Long, Liu et al. 2006; Raghunandan, Agrawal et al. 2010) support our findings that vaginal atrophy and sexual dysfunction are two separate entities which are both, at least partially, secondary to sex steroid deficiency. Women receiving hormone therapy have been shown to have overall reduction of vaginal innervation, estimated by parasympathetic, sympathetic and sensory axons. The effect was more pronounced in women receiving intravaginal estrogen therapy (Griebling, Liao et al. 2012). While the authors have suggested that the estrogen-induced reduction in vaginal innervation may be related to a relief of vaginal discomfort, such data certainly do not suggest an increase in nerve sensitivity or density as found after testosterone (Pessina, Hoyt et al. 2006) or prasterone (Pelletier, Ouellet et al. 2012a; Pelletier, Ouellet et al. 2012b) treatment in rats.

Sex steroid deficiency is the equivalent or a consequence of DHEA deficiency (Labrie 2010a). Some of the disorders of postmenopause are more directly related to a lack of estrogens (ex. vaginal atrophy, hot flushes, ...) while others are more related to defective androgenic activity (ex. muscle mass and strength, sexual dysfunction, ...). Recent data have shown the benefits of the local intravaginal action of DHEA on the four domains of sexual dysfunction (Labrie, Archer et al. 2009b), while the symptoms of vaginal atrophy were also markedly improved (Labrie, Archer et al. 2009b). Since it is recognized, as mentioned above, that estrogens improve vaginal atrophy symptoms by an action in the most superficial layer of the vagina without affecting sexual dysfunction (Lobo, Rosen et al. 2003; Gonzalez, Viafara et al. 2004; Long, Liu et al. 2006; Raghunandan, Agrawal et al. 2010) while DHEA affects both vaginal atrophy and sexual dysfunction, it appears logical to conclude that vaginal atrophy and sexual dysfunction (called VVSD, vulvovaginal sexual dysfunction) are two separate medical entities in analogy with osteoporosis and hot flushes which are two different medical entities both secondary to a lack of sex steroids due to low DHEA activity, DHEA being the only source of sex steroids after menopause.

Such an interpretation is well supported by the preclinical observation of a specific and marked stimulatory effect of androgens on nerve fibers (Pessina, Hoyt et al. 2006) and collagen (Berger, El-Alfy et al. 2005) in the lamina propria, the intermediate layer of the vagina, while vaginal atrophy is typically a problem of the external epithelial layer, a tissue responsive to estrogens but minimally responsive to androgens (Berger, El-Alfy et al. 2005; Pessina, Hoyt et al. 2006). The morphological changes observed in the vagina after DHEA treatment reflects its local conversion into active sex steroids having androgenic and/or estrogenic action through intracrine mechanisms (Labrie 1991). The changes observed in the rat include epithelial mucification, high compactness of delicate, finely woven lamina propria collagen fibers and moderate muscularis thickness increase when compared to ovariectomized (OVX) animals. These morphological changes are typical of androgenic effects. In the lamina propria, fine newly synthesized collagen fibers are seen near the epithelium. Labeling of the androgen receptor is increased by about 3-fold in the three vaginal layers (epithelium, lamina propria and muscularis) after DHEA administration (Berger, El-Alfy et al. 2005).

Of particularly high relevance to sexual dysfunction is the observation that nerve fibers are mainly located in the lamina propria, a main site of action of androgens, and that treatment with testosterone increases the number and size of the nerve terminals in the lamina propria while estrogens and progesterone have no effect (Pessina, Hoyt et al. 2006). It is of particular interest to mention that a stimulatory effect of prasterone on intravaginal nerve density has recently been observed (Pelletier, Ouellet et al. 2012a). The effect of prasterone was not influenced by simultaneous treatment with the pure antiestrogen acolbifene, thus indicating an androgenic action of prasterone (Pelletier, Ouellet et al. 2012b). In agreement with (Pessina, Hoyt et al. 2006), no effect of estrogens was observed on the density of the vaginal nerves (Pelletier, Ouellet et al. 2012b). Such data strongly suggest that the action of DHEA on sexual dysfunction is a specific androgenic effect secondary to the conversion of DHEA into androgens in the nerve fibers of the vagina. Increased sensitivity of the vaginal nerve fibers by the androgenic component of DHEA action is the most likely explanation for the beneficial effects observed on sexual dysfunction in women receiving intravaginal DHEA (Labrie, Archer et al. 2009a).

In terms of mechanism of action, it is important to remember that low libido and low coital frequency was not affected in postmenopausal women who received oral or percutaneous estrogen (Long, Liu et al. 2006) even if a significant effect was observed on vaginal dryness and pain at intercourse, thus indicating a dissociation between the effect of estrogen on vaginal atrophy and sexual dysfunction. Similar findings have been reported by (Lobo, Rosen et al. 2003; Gonzalez, Viafara et al. 2004). In patients with both vaginal atrophy and FSD, who were treated for 12 weeks with Premarin cream, Premarin + testosterone cream or placebo, an improvement in the sexuality score was observed only in the Premarin + testosterone group while vaginal atrophy was improved in both the Premarin and Premarin + testosterone groups (Raghunandan, Agrawal et al. 2010).

The data showing that estrogens correct vaginal atrophy symptoms without significantly affecting sexual dysfunction clearly indicate that vaginal atrophy and sexual dysfunction are two separate entities which are both, at least partially due to sex steroid deficiency. As mentioned above, sexual dysfunction can then be considered a member of the spectrum of symptoms and medical problems related to sex steroid deficiency in postmenopausal women, namely osteoporosis, muscle loss, skin atrophy, type 2 diabetes, fat accumulation, vaginal atrophy, sexual dysfunction, memory loss, cognition loss and possibly Alzeiheimer's disease (Labrie 2007; Labrie 2010a). In fact several studies have reported diminished sexual desire or interest, decreased sexual receptivity and decreased sexual responsiveness in postmenopausal women (Hallstrom 1977; Osborn, Hawton et al. 1988; Hallstrom and Samuelsson 1990; Dennerstein, Smith et al. 1994; Bancroft and Cawood 1996; Avis, Stellato et al. 2000; Dennerstein, Dudley et al. 2001).

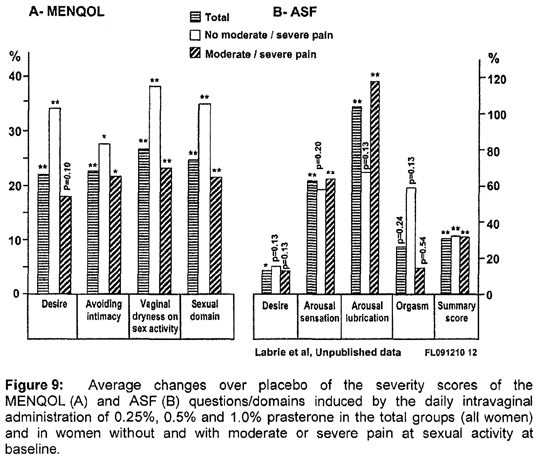

On the other hand, approximately 25% of postmenopausal women with one or more moderate or severe (MS) symptom(s) of vaginal atrophy do not have MS pain at sexual activity. The presence or absence of MS pain at baseline, however, has no influence on the improvement over placebo of desire and arousal by intra-vaginal administration of DHEA (Tables 1 and 2; Figure 9).

While any beneficial effect of intravaginal DHEA on FSD was believed to be secondary to the treatment effects on vulvovaginal atrophy symptoms, with secondary improvement of sexual dysfunction, the treatment with local intra-vaginal DHEA surprisingly had a positive effect on female sexual function with no influence of the presence or absence of moderate to severe pain at sexual activity or dyspareunia at baseline (see Figures 9 and 11). In fact, the effect on female sexual function was found to be independent of treatment effects on dyspareunia. Hence, intravaginal DHEA could be an effective treatment of symptoms of FSD in women irrespective of the presence of vulvovaginal atrophy symptoms. Since sexual dysfunction appears to be secondary to a decrease in DHEA activity and that the decrease in DHEA activity starts much earlier than menopause (Labrie 1991; Labrie, Luu-The et al. 2005; Labrie 2010a; Labrie 2010b), the present findings of the absence of influence of dyspareunia on the benefits of DHEA on sexual dysfunction, suggest that DHEA could be used successfully to treat sexual dysfunction in premenopausal women in the absence of VVA symptoms. In fact, practically all androgens in women derive from the transformation of DHEA into testosterone and dihydrotestosterone (DHT) in peripheral tissues in both pre- and postmenopause (Labrie, Martel et al. 2011). Moreover, serum DHEA levels start decreasing by the age of 30 years with a 60% loss already observed at time of menopause with no significant contribution of the ovary (Labrie 1991; Labrie, Luu-The et al. 2005; Labrie, Bélanger et al. 2006). It is thus logical that sexual dysfunction observed in premenopausal women be caused by a lack of DHEA activity. Since the effect of sex steroids on sexual dysfunction appears to be due to androgens, the present findings of a similar beneficial effect of prasterone in women having or not having moderate to severe dyspareunia strongly support this conclusion and indicate that sexual dysfunction related to a lack of androgens in premenopausal women is due to a lack of DHEA activity, the only source of androgens which starts to decrease at the age of 30 years.

**DHEA** as mentioned in the claims stands for (3β)-3-Hydroxyandrost-5-en-17-one (also known as 5-Dehydroepiandrosterone or prasterone) and its pharmaceutically acceptable forms, especially salts. DHEA has the formula:

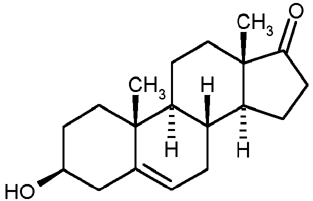

It was first described after isolation from male urine [Butenandt A. and Tscherning K., Z. Physiol. Chem. 229, 167 (1934); Butenandt and Dannenbaum, Z. Physiol. Chem. 229, 192 (1934); preparation from cholesterol: Butenandt A.et al., Z. Physiol. Chem. 237, 57 (1935)].

**Intravaginal DHEA** means DHEA locally administered in the vagina, e.g. with intravaginal ovules, suppositories (as described in WO 09/21323), creams, lotions, gels, ointments, rings and the like.

**Hypoactive Sexual Desire Disorder** (HSDD) as mentioned in the claims means bothersome lack of interest in engaging in sexual activity.

**Female Sexual Arousal Disorder** (FSAD) as mentioned in the claims means bothersome lack of becoming aroused despite desire to engage in sexual activity. **Female Orgasmic Disorder** (FOD) as mentioned in the claims means bothersome failure to reach orgasm despite arousal.

These disorders are further defined in the Diagnostic and Statistical Manual of Mental Disorders, Fourth Edition (DSM-IV). These definitions together with diagnostic criterias can be also found under:
www.behavenet.com/capsules/disorders/hyposexdesdis.htm (HSDD),
www.behavenet.com/capsules/disorders/fsexarsldis.htm (FSAD) and
www.behavenet.com/capsules/disorders/forgdis.htm (FOD).

**Female Interest Arousal Disorder** (FIAD) is the simultaneous presence of desire and arousal disorders.

Menopause follows the last natural, ovarian-induced menstrual period as a result of a permanent cessation of the ovarian cycle and any significant hormonal activity of the ovaries (Labrie, Martel et al. 2011) and is declared in retrospect after amenorrhoea of one complete year. **Postmenopausal women** are thus adult women beyond menopause, i.e. women that have entered the time of their life that takes place after their last period, or more accurately, all of the time that follows the point when their ovaries become inactive.

**Vulvar and vaginal atrophy** (VVA) is a condition resulting from hormone deficiency after the menopause that is associated with typical symptoms that may include vaginal dryness, burning and itching and vaginal pain during sexual activity and the findings of a friable or irritated vaginal and vulvar surface with thin vaginal epithelium showing a maturation index with a high ratio of parabasal to superficial epithelial cells (less than 5% superficial cells) and a vaginal pH > 5.0.

### EXAMPLE

The example below is intended only to illustrate a particular embodiment of the present invention and is not meant to limit the scope of the invention in any manner.

This study (ERC-210) is a phase III, prospective, multicentre, randomized, placebo-controlled, and double-blind trial. The original intend-to-treat (ITT) population included 216 postmenopausal women randomized to receive a daily ovule of the following DHEA concentrations: 0.0% (53 women), 0.25% (3.25 mg DHEA, 53 women), 0.5% (6.5 mg DHEA, 56 women) or 1.0% (13 mg DHEA, 54 women) administered intravaginally with an applicator at bedtime for 12 weeks (Labrie, Archer et al. 2009b). Of this population, one woman did not have data for the sexual function parameters at baseline, thus reducing the total number of patients to 215. The DHEA ovules or suppositories (VaginormTM) containing prasterone in a lipophilic base were manufactured by Recipharm, Karlskoga, Sweden. The study was divided into two phases, namely screening followed by a treatment period of 12 weeks. The protocol was approved by the Institutional Review Board of the Centre Hospitalier de l'Université Laval (Quebec City, Canada), McGill University (Montreal, Canada), Ethica (Montreal, Canada), Eastern Virginia Medical School (Norfolk, Virginia, USA) and the Western Institutional Review Board (Los Angeles, USA).

The inclusion and exclusion criteria were as described in (Labrie, Archer et al. 2009b). A written informed consent was obtained from all subjects prior to the performance of any study-related procedure. The subjects had a medical history, a medical examination and a complete gynecological examination at screening. A partial gynecological examination was performed to evaluate the aspect of the mucosa and tolerance to the medication on day 1 and at all visits. The standard laboratory tests, namely hematology (including complete blood count and coagulation), blood chemistry and urinalysis were performed at screening, day 1 and at all visits. Summary tabulations were prepared displaying the number of observations, mean or geometric mean as appropriate, SD; SEM, 95% two-sided CI; median, minimum, and maximum for continuous variables; and the number and percentage per category for categorical data. Statistical analyses were performed at the two-sided significance level of 0.05 unless otherwise stated. The categories for summarization in general consisted of the dose levels of the DHEA treatments, 0% (placebo), 0.25%, 0.5% and 1.0% DHEA. The endpoints for analysis consisted of the following: desire/interest, arousal and orgasm were self-rated by the women at screening, at day 1, and at week 4, 8 and 12 using the ASF and MENQOL questionnaires. Baseline was defined as the value on day 1, obtained before first use of the study treatment, and change from baseline to week 12 was calculated for each participant for each domain endpoint. The analysis comparing each dose of DHEA to placebo was performed by analysis of covariance, with the baseline value used as the covariate. Missing values were replaced by the last observation made.

The three questions covered by the sexual domain of the MENQOL questionnaire are change in sexual desire, intimacy avoidance and vaginal dryness during sexual activity. In our previous publication (Labrie, Archer et al. 2009a), only women having a score value at baseline and week 12 were used in the calculations while the present evaluation carries forward the last value obtained after baseline when data at week 12 are missing. More importantly, the present analysis is made separately for women who had moderate or severe (MS) pain at sexual activity at baseline and for women who did not have MS pain at baseline, in order to detect a possible difference between the two groups in terms of response. Since the number of women is as low as 12 in some groups for some parameters after the distribution is made between women with and without MS, the calculations have also been performed with the combination of the three prasterone doses, although the number of women remains low in the corresponding placebo group.

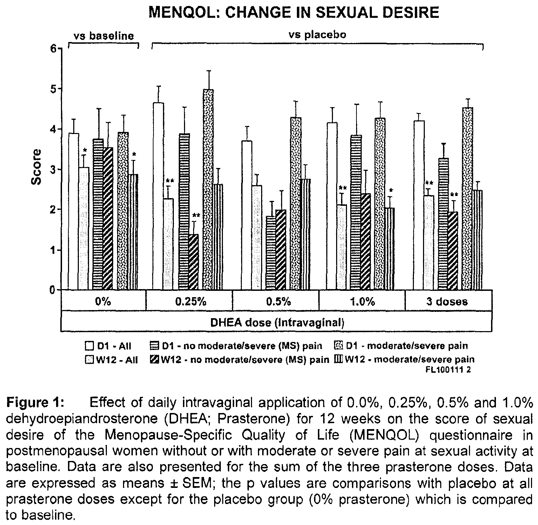

As can be seen in Table 1 and Figure 1, sexual desire is improved at week 12 by 22% (p=0.016), 51% (p=0.0047), 31% (p=0.2845) and 48% (p=0.0072) in the placebo, 0.25%, 0.5% and 1.0% DHEA groups, respectively. The p values for the DHEA groups are always for values compared to placebo while the placebo group is always compared to baseline in this and the following figures. Fifty three to fifty six women are evaluable in each group for the ITT analysis. It can also be seen in Table 1 and Figure 1 that when the 53 placebo-treated women are compared to the group of all women (n=163) who received prasterone (0.25%, 0.5% or 1.0%), the score of sexual desire is improved by 44% in the prasterone group (p=0.0083 compared to the placebo effect).

As shown in Table 1, from 12 to 16 women are evaluable in the groups with no MS pain at sexual activity at baseline and 37 to 43 women in the groups with MS pain. There are thus approximately 25% of women having no MS pain compared to about 75% of women having MS pain at baseline. Despite the small number of women in the no MS pain, it can be seen in Table 1 and Figure 1, that sexual desire is improved by 6.1% (p=0.4890), 61% (p=0.0002), -8.1% (p=0.3158) and 35% (p=0.0551) in the 0%, 0.25%, 0.5% and 1.0% in the no MS pain DHEA groups, respectively. The improvements in the corresponding MS pain groups are 27% (p=0.0154), 48% (p=0.1746), 36% (p=0.5876) and 53% (p=0.0332). When all the prasterone-treated women are compared to placebo, a 40.2% increase (p=0.0073) in sexual desire is observed in the no MS pain group while a 45% increase (p=0.1058) is observed in the group of women with MS pain at baseline.

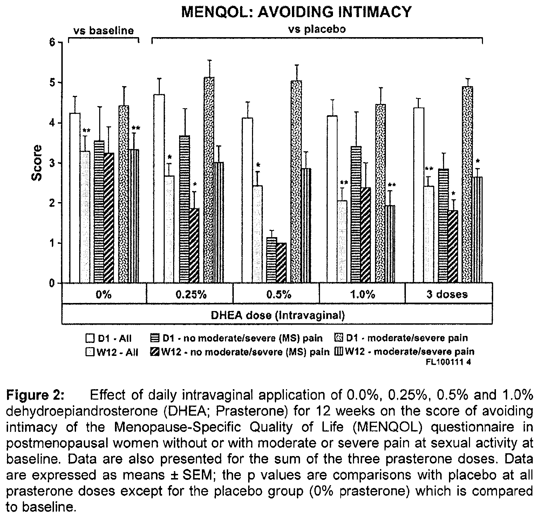

When the second question of the MENQOL is considered, namely avoiding intimacy, decreases in the score of 22% (p=0.0072), 43% (p=0.0411), 41% (p=0.0431) and 50% (p=0.0038) are observed in the 0%, 0.25%, 0.5% and 1.0% DHEA total groups, respectively (Table 1 and Figure 2). When the prasterone-treated groups are combined, the severity of the score avoiding intimacy is improved by 44.8% (p=0.0047) (Figure 2, Table 1). For the no MS pain groups, changes of 9.2% (p=0.6575), 49% (p=0.0233), 13% (p=0.0502; NS versus baseline) and 27% (p=0.2715) are observed in the corresponding groups. In the MS pain at sexual activity groups, on the other hand, improvements of 25% (p=0.0051), 41% (p=0.2215), 43% (p=0.1463) and 56% (p=0.0064) are observed with the corresponding DHEA doses. When placebo is compared to the group of all prasterone-treated women, improvements of 36.6% (p=0.0313) and 46.4% (p=0.0271) are observed in the no MS and MS pain groups, respectively.

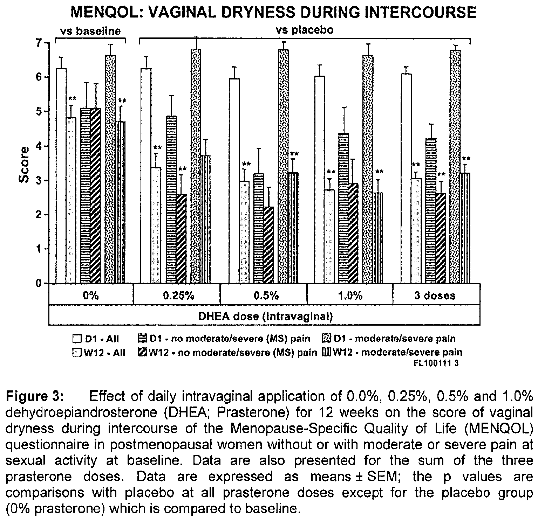

When the third question of the MENQOL is considered, namely vaginal dryness or lubrication during intercourse, it can be seen in Table 1 and Figure 3 that in the different groups, vaginal dryness improved at 12 weeks compared to baseline by 23% (p=0.0004), 45% (p=0.0032), 50% (p=0.0003) and 54% (p<0.0001) in the 0%, 0.25%, 0.5% and 1.0% DHEA groups, respectively. When analyzed for the total prasterone group, the vaginal dryness score is improved by 49.9% (p<0.0001 compared to placebo). In the corresponding no MS pain groups, vaginal dryness improved by 0% (p=1.00), 47% (p=0.0032), 31% (p=0.0234; NS versus baseline) and 31% (p=0.0357; NS versus baseline). In the MS groups, on the other hand, decreases of 29% (p<0.0001), 45% (p=0.070), 52% (p=0.0056) and 60% (p=0.0004) were observed in the same treatment groups. When women of the 3 prasterone doses are combined in the no MS and MS pain groups, improvements of 38.2% (p=0.0028) and 52.4% (p=0.0009) are observed, respectively.

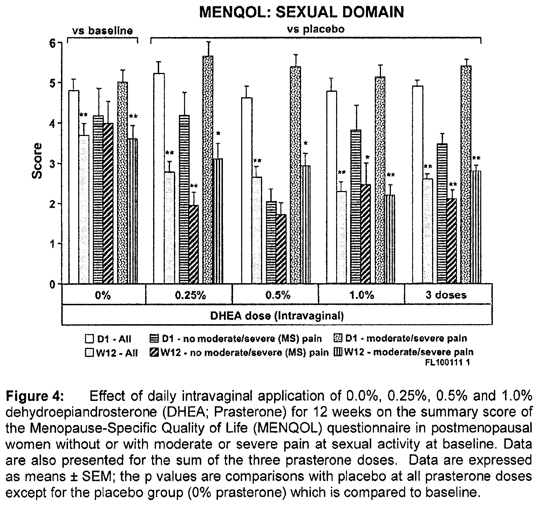

It is of interest to observe that following combination of the three sexual function - related questions of the MENQOL (sexual domain), improvements of 22% (p=0.0001), 47% (p=0.0003), 42% (p=0.0022) and 51% (p<0.0001) are observed in the 0%, 0.25%, 0.5% and 1.0% DHEA groups, respectively (Table 1, Figure 4). Combining the women who received the different prasterone doses leads to a 46.7% improvement of the severity score of the sexual domain (p<0.0001 compared to placebo). When the groups with no MS pain are analyzed, decreases of 4.3% (p=0.6944), 53% (p<0.0001), 16% (p=0.0194) and 31% (p=0.0176) are observed in the corresponding groups while improvements of 27% (p<0.0001), 45% (p=0.0298), 45% (p=0.0203) and 57% (p=0.0001) are observed in the MS pain groups. When women without and with MS pain are analyzed separately, the severity scores are improved by 39.2% (p=0.0003) and 48.4% (p=0.00007), respectively.

It can also be seen in Table 1 that the severity of the summary score of the MENQOL questionnaire is improved by 20.8% (p=0.0023, compared to placebo) when the group of all women treated with prasterone is considered. In the no MS and positive MS pain groups, respective improvements of the summary score of 18% (p=0.0475) and 21.4% (p=0.0148) are observed.

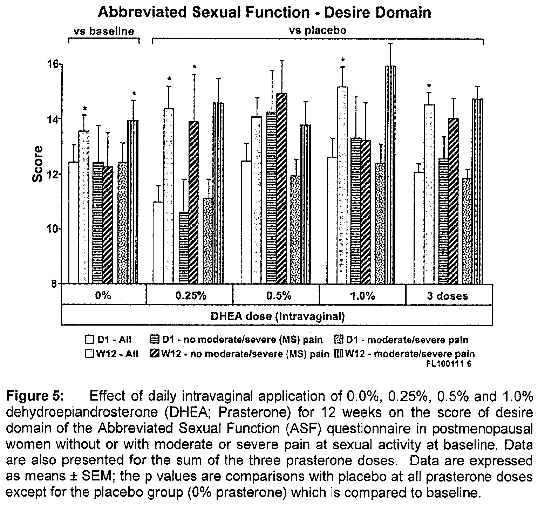

The other questionnaire used in our study to investigate sexual dysfunction has been the Abbreviated Sexual Function (ASF) questionnaire. When the desire domain of ASF is analyzed, it can be seen in Table 2 and Figure 5 that improvements of 8.7% (p=0.0458), 31% (p=0.0260), 13% (p=0.5193) and 22% (p=0.0322) were observed in the 0%, 0.25%, 0.5% and 1.0% DHEA groups, respectively. On the other hand, the group of all women treated with intravaginal prasterone shows a 21.0% improvement of the ASF desire domain (p=0.0436). When the groups with no MS pain are considered, improvements of -1.2% (p=0.9028), 31% (p=0.0337), 4.8% (p=0.3706) and 3.7% (p=0.5372) are observed compared to 12% (p=0.0134), 32% (p=0.1349), 16% (p=0.8546) and 29% (p=0.0341) in the corresponding MS pain groups. Corresponding improvements of 14.0% (p=0.1273) and 24.9% (p=0.1312) are observed in the no MS and MS pain groups for all women treated with prasterone versus placebo.

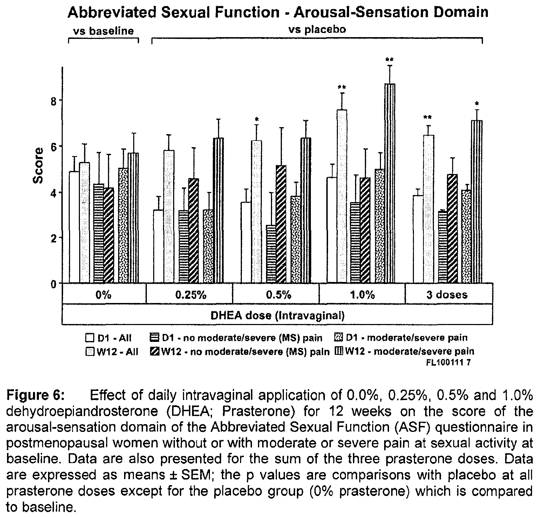

When the arousal-sensation domain of ASF is analyzed (Table 2, Figure 6), improvements of 8.6% (p=0.5584), 80% (p=0.0673), 72% (p=0.0498) and 64% (p=0.0039) are observed in the total groups treated with 0%, 0.25%, 0.5%, and 1.0% DHEA, respectively. A 71.2% improvement (p=0.0064) of the arousal/sensation domain is observed when all women treated with prasterone are considered. When women with no MS pain are analyzed, changes of -5.3% (p=0.8912), 43% (p=0.3539), 103% (p=0.1137) and 30% (p=0.4486) are observed compared to 12% (p=0.4244), 97% (p=0.1024), 66% (p=0.1623) and 72% (p=0.0023) in the corresponding MS pain groups. When the three groups of women treated with prasterone are combined and compared to the corresponding placebo groups, improvements of 52.7% (p=0.1963) and 76.2% (p=0.0132) are observed for the women without and with MS pain, respectively.

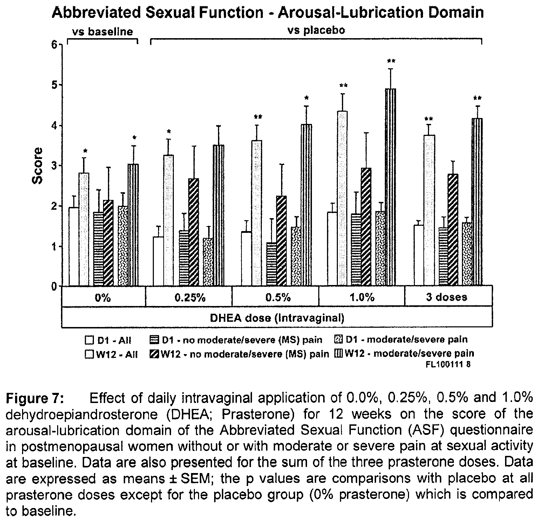

When the arousal-lubrication domain is analyzed, changes of 45% (p=0.0183), 159% (p=0.0233), 159% (p=0.0058) and 135% (p=0.0007) are observed for the total groups of women treated with 0%, 0.25%, 0.5% and 1.0% DHEA, respectively (Table 2 and Figure 7). A 149% improvement (p=0.0006) of the ASF sensation/lubrication score is obtained for the combined prasterone-treated groups of women are compared to placebo. When women with no MS pain are analyzed, improvements of 17% (p=0.6465), 95% (p=0.1521), 106% (p=0.2200) and 63% (p=0.2755) are observed compared to 53% (p=0.0172), 189% (p=0.0556), 170% (p=0.0129) and 162% (p=0.0007) for the corresponding groups with MS pain at baseline. When the groups of all prasterone-treated women are compared to placebo, respective improvements of 84.6% (p=0.1254) and 171% (p=0.0014) are observed for the women without and with MS pain at baseline.

When the orgasm domain of the ASF questionnaire is analyzed, a strong placebo effect is observed in response to the composite of the three following questions; a) over the last 4 weeks, how often did you have an orgasm when you took part in sexual activity (may be with or without partner); b) over the last 4 weeks, in general, how pleasurable were the orgasms the you had and; c) over the last 4 weeks, in general, how easy was it for you to reach orgasm? With the relatively small number of subjects in each group, no statistical significance was reached in any group despite a p value of 0.0838 for the total group of women treated with 1.0% DHEA and a p value of 0.0737 for the group of women with no MS pain at baseline treated with 0.5% DHEA (Table 2). When the combined three groups of prasterone-treated women are compared to placebo, improvements of 62.4% (p=0.2378), 55.3% (p=0.1297) and 64.3% (p=0.5424) are observed for the total, no MS pain and MS pain groups, respectively.

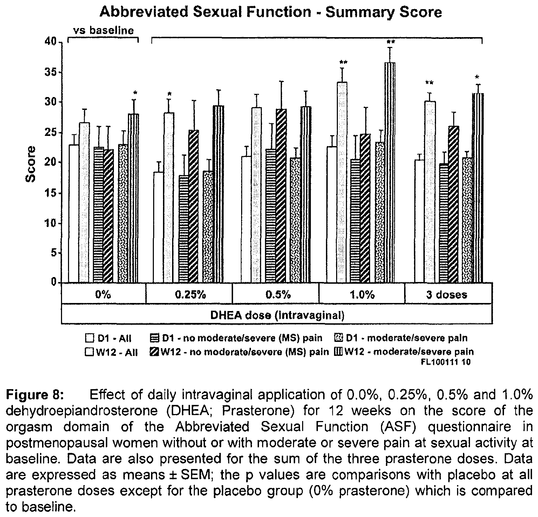

When the summary scores of the ASF are analyzed in the total groups, the improvements observed are 16% (p=0.0563), 54% (p=0.0445), 39% (p=0.0984) and 48% (p=0.0047) in the 0%, 0.25%, 0.5% and 1.0% groups, respectively (Table 2 and Figure 8). Comparison of the combination of the three groups of prasterone-treated women to placebo shows an improvement of 46.6% (p=0.0083) of the summary score. In the no MS pain groups, the changes are -1.0% (p=0.9567), 42% (p=0.0881), 31% (p=0.1149) and 21% (p=0.3073) while changes of 21% (p=0.0226), 59% (p=0.1399), 42% (p=0.3085) and 57% (p=0.0046) are observed in the MS pain groups. On the other hand, improvements of 31.1% (p=0.0807) and 52.1% (p=0.0306) are seen in the combined groups do DHEA-treated women having no or MS pain at baseline, respectively (Table 2).

When the percentages of changes from baseline in the sexual domain (sum of desire, avoiding intimacy and vaginal dryness) following the three DHEA doses are combined in the MENQOL questionnaire, average percentages of change of 33.3% and 49.0% are observed in the non MS and MS pain groups, respectively (Table 1). When the differences from placebo are considered, thus removing the placebo effect, the average percentage of changes following combination of the three DHEA groups are 34.9% (p<0.01) and 21.4% (p<0.01), respectively, for the no MS and MS pain groups, with a 24.7% (p<0.01) change in the total group combining women with and without MS pain at baseline (Table 3). When the same analysis is made with the summary score of the ASF questionnaire, the differences from placebo are 32.1% (N.S.) and 31.7% (p<0.05), in the non MS and MS pain at sexual activity groups, respectively, with a 31.0% (p<0.01) change in the total group (Table 3). Except for the orgasm of the ASF questionnaire where the precision is too low to make meaningful conclusions, it can be seen in Table 3 that for all questions of the MENQOL and for all domains of the ASF, there is no indication of a difference in response over placebo between women having or not having MS pain at sexual activity at baseline. These comparisons can be more easily seen on Figure 9.

As can already be seen in Tables 1 and 2, for all the parameters studied in both the MENQOL and ASF questionnaires, the placebo effects observed in the total groups are practically completely attributable to the women having MS pain at sexual activity at baseline. In fact, for the sexual domain of the MENQOL questionnaire (questions 27, 28 and 29), the global placebo effect is 4.3% in the group of women having no MS pain at baseline compared to 27% in women having MS pain (Table 1, Figure 10A). For the ASF summary score, the global placebo effects are calculated at -1.0% and 21%, respectively, in the no MS and MS pain groups (Table 2; Figure 10B). As can also be seen in Figure 10, the same observation of the placebo effect being present almost exclusively in women with MS pain at baseline applies to all individual questions of the MENQOL and all domains of the ASF.

It is then of interest to investigate the possibility that the severity score of sexual dysfunction at baseline could be different in the two different groups of women, namely those without and those with MS pain at sexual activity at baseline. As can be seen in Table 4, the average scores for sexual desire in the MENQOL questionnaire were 3.34 and 4.38 in the groups of women without and with MS pain at sexual activity, respectively. In the same groups, the average scores for avoiding intimacy were 2.96 and 4.78 respectively, while respective values of 4.42 and 6.76 were observed for vaginal dryness at sexual activity. The severity scores of the total sexual domain of MENQOL were measured at 3.60 and 5.31 in women without and with MS pain at sexual activity, respectively.

For the ASF questionnaire, the average severity scores for sexual desire, arousal/sensation, arousal/lubrication and orgasm were 12.69 and 12.02 (maximum=30), 3.41 and 4.28 (maximum=20), 1.53 and 1.64 (maximum=10) as well as 3.29 and 3.46 (maximum=15) in the groups of women without and with MS pain at baseline, respectively. For the summary score, values of 20.69 and 21.39 were observed in the same groups (Table 5).

Since a potentially large placebo effect is a general problem for studies on sexual dysfunction, it is of interest to compare directly on the same graph (Figure 11A) the changes observed for the placebo effects as well as the changes over placebo in the desire, avoiding intimacy, vaginal dryness and total sexual domains evaluated by the MENQOL questionnaire. Most interestingly, the major difference between the no MS and MS pain groups is, as mentioned above, in the placebo effects. In fact, total changes in the placebo effects (sum of the three questions of the MENQOL) are calculated from Table 1 at 15.3% and 81.0% in the no MS and MS pain groups, respectively, for a 5.3-fold higher placebo effect in the women having MS pain compared to no MS pain at baseline.

As can be seen in Table 3, the changes over placebo (same three questions of the MENQOL) in the sexual function domain of the MENQOL are 99.7% and 62.8%, respectively, for the no MS and MS pain groups, respectively. As a consequence, the specific (above placebo) effects in the MENQOL questionnaire show global 5.5- and 0.78-fold increases over placebo in the non MS and MS pain groups, respectively, thus indicating a 6.8-fold higher specificity of correction of sexual dysfunction (over placebo) in the women with no MS pain at baseline.

As illustrated in Figure 11B, comparable findings are observed in the ASF questionnaire where the sums of placebo changes for the non MS and MS groups are 6.4% and 127%, respectively (Table 2), while the sum of changes above placebo in the corresponding groups are 200.2% and 209.4%, respectively (Table 3). It is clear from the present data that a much higher change relative to placebo is observed in the non MS group where only a 6.4% placebo effect is observed in the ASF questionnaire while the specific (above placebo) effect is 202.2% for a 31.6-fold increase over placebo (3060% increase). In the MS group, on the other hand, the placebo and over placebo changes are 127% and 209.4%, respectively, for a much lower 1.6-fold (65%) increase over placebo. The specificity of the effect of DHEA in the non MS pain group of women is thus 30-fold higher than in the group of women having moderate to severe (MS) pain at baseline

### REFERENCES

American Psychiatric Association (2000). Diagnostic and Statistical Manual of Mental Disorders (DSM-IV-TR), Washington, DC, Chapter 11, Sexual and Gender Identity Disorders.
Avis, N. E., R. Stellato, et al. (2000). "Is there an association between menopause status and sexual functioning?" Menopause 7(5): 297-309.
Bancroft, J. and E. H. Cawood (1996). "Androgens and the menopause; a study of 40-60-year-old women." Clin Endocrinol (Oxf) 45(5): 577-587.
Basson, R. (2007). "Hormones and sexuality: current complexities and future directions." Maturitas 57(1): 66-70.
Basson, R., L. A. Brotto, et al. (2010). "Role of androgens in women's sexual dysfunction." Menopause 17(5): 962-971.
Basson, R., R. McInnes, et al. (2002). "Efficacy and safety of sildenafil citrate in women with sexual dysfunction associated with female sexual arousal disorder." J Womens Health Gend Based Med 11(4): 367-377.
Béjin, A. (1994). Sexual pleasures, dysfunctions, fantaisies, and satisfaction.. A. Spira, Bajos, N. eds. Sexual Behaviour and AIDS. Aldershot, England: Avebury.: 163-171.
Berger, L., M. El-Alfy, et al. (2005). "Effects of dehydroepiandrosterone, Premarin and Acolbifene on histomorphology and sex steroid receptors in the rat vagina." J Steroid Biochem Mol Biol 96(2): 201-215.
Berman, J. R., L. A. Berman, et al. (2003). "Safety and efficacy of sildenafil citrate for the treatment of female sexual arousal disorder: a double-blind, placebo controlled study." J Urol 170(6 Pt 1): 2333-2338.
Burwell, S. R., L. D. Case, et al. (2006). "Sexual problems in younger women after breast cancer surgery." J Clin Oncol 24(18): 2815-2821.
Caruso, S., G. Intelisano, et al. (2001). "Premenopausal women affected by sexual arousal disorder treated with sildenafil: a double-blind, cross-over, placebo-controlled study." Biog 108(6): 623-628.
Caruso, S., S. Rugolo, et al. (2006). "Sildenafil improves sexual functioning in premenopausal women with type 1 diabetes who are affected by sexual arousal disorder: a double-blind, crossover, placebo-controlled pilot study." Fertil Steril 85(5): 1496-1501.
Davis, S. R., S. L. Davison, et al. (2005). "Circulating androgen levels and self-reported sexual function in women." JAMA 294(1): 91-96.
Davis, S. R., P. McCloud, et al. (1995). "Testosterone enhances estradiol's effects on postmenopausal bone density and sexuality." Maturitas 21(3): 227-236.
Davis, S. R. and J. Tran (2001). "Testosterone influences libido and well being in women." Trends Endocrinol Metab 12(1): 33-37.
Dennerstein, L., E. Dudley, et al. (2001). "Are changes in sexual functioning during midlife due to aging or menopause?" Fertil Steril 76(3): 456-460.
Dennerstein, L., A. M. Smith, et al. (1994). "Sexuality and the menopause." J Psychosom Obstet Gynaecol 15(1): 59-66.
Goldstat, R., E. Briganti, et al. (2003). "Transdermal testosterone therapy improves well-being, mood, and sexual function in premenopausal women." Menopause 10(5): 390-398.
Gonzalez, M., G. Viafara, et al. (2004). "Sexual function, menopause and hormone replacement therapy (HRT)." Maturitas 48(4): 411-420.
Griebling, T. L., Z. Liao, et al. (2012). "Systemic and topical hormone therapies reduce vaginal innervation density in postmenopausal women." Menopause 19(6): 630-635.
Hallstrom, T. (1977). "Sexuality in the climacteric." Clin Obstet Gynaecol 4(1): 227-239.
Hallstrom, T. and S. Samuelsson (1990). "Changes in women's sexual desire in middle life: the longitudinal study of women in Gothenburg." Arch Sex Behav 19(3): 259-268.
Hayes, R. D., L. Dennerstein, et al. (2008). "Risk factors for female sexual dysfunction in the general population: exploring factors associated with low sexual function and sexual distress." J Sex Med 5(7): 1681-1693.
Hubayter, Z. and J. A. Simon (2008). "Testosterone therapy for sexual dysfunction in postmenopausal women." Climacteric 11(3): 181-191.
Hulter, B. and P. O. Lundberg (1994). "Sexual function in women with hypothalamo-pituitary disorders." Arch Sex Behav 23(2): 171-183.
Kontula, O. and E. Haavio-Mannila (1995). "Sexual Pleasures. Enhancement of Sex Life in Finland, 1971-1992. Aldershot, England: Dartmouth."
Labrie, F. (1991). "Intracrinology." Mol. Cell. Endocrinol. 78: C113-C118.
Labrie, F. (2001). "Dehydroepiandrosterone (DHEA) as potential hormone replacement therapy." Référence en Gynécologie Obstétrique 8: 317-322.
Labrie, F. (2007). "Drug Insight: breast cancer prevention and tissue-targeted hormone replacement therapy." Nature Clinical Practice, Endocrinology & Metabolism. 3(8): 584-593.
Labrie, F. (2010a). "DHEA after Menopause - Sole source of sex steroids and potential sex steroid deficiency treatment." Menopause Management 19: 14-24.
Labrie, F. (2010b). DHEA, important source of sex steroids in men and even more in women. Neuroendocrinology, The Normal Neuroendocrine System, Progress in Brain Research. L. Martini, Chrousos G.P, Labrie F, Pacak K and D. Pfaff, eds., Elsevier. 182 (Chapter 4): 97-148.
Labrie, F., D. Archer, et al. (2009a). "Effect on intravaginal dehydroepiandrosterone (Prasterone) on libido and sexual dysfunction in postmenopausal women." Menopause 16: 923-931.
Labrie, F., D. Archer, et al. (2009b). "Intravaginal dehydroepiandrosterone (Prasterone), a physiological and highly efficient treatment of vaginal atrophy." Menopause 16: 907-922.
Labrie, F., D. Archer, et al. (2009c). "Serum steroid levels during 12-week intravaginal dehydroepiandrosterone administration." Menopause 16: 897-906.
Labrie, F., A. Bélanger, et al. (2006). "Androgen glucuronides, instead of testosterone, as the new markers of androgenic activity in women." J Steroid Biochem Mol Biol 99: 182-188.
Labrie, F., L. Cusan, et al. (2008a). "Corrigendum to: Effect of intravaginal DHEA on serum DHEA and eleven of its metabolites in postmenopausal women." J Steroid Biochem Mol Biol 112: 169.
Labrie, F., L. Cusan, et al. (2008b). "Effect of Intravaginal DHEA on Serum DHEA and Eleven of its Metabolites in Postmenopausal Women." Journal Ster Biochem & Mol Biol 111: 178-194.
Labrie, F., P. Diamond, et al. (1997). "Effect of 12-month dehydroepiandrosterone replacement therapy on bone, vagina, and endometrium in postmenopausal women." J Clin Endocrinol Metab 82(10): 3498-3505.
Labrie, F., V. Luu-The, et al. (2005). "Is DHEA a hormone? Starling Review." J Endocrinol 187: 169-196.
Labrie, F., V. Luu-The, et al. (2003). "Endocrine and intracrine sources of androgens in women: inhibition of breast cancer and other roles of androgens and their precursor dehydroepiandrosterone." Endocrine Reviews 24(2): 152-182.
Labrie, F., C. Martel, et al. (2011). "Wide distribution of the serum dehydroepiandrosterone and sex steroid levels in postmenopausal women: role of the ovary?" Menopause 18(1): 30-43.
Lasco, A., N. Frisina, et al. (2001). "Metabolic effects of dehydroepiandrosterone replacement therapy in postmenopausal women." Eur J Endocrinol 145: 457-461.
Laumann, E. O., A. Paik, et al. (1999). "Sexual dysfunction in the United States: prevalence and predictors." Jama 281(6): 537-544.
Lobo, R. A., R. C. Rosen, et al. (2003). "Comparative effects of oral esterified estrogens with and without methyltestosterone on endocrine profiles and dimensions of sexual function in postmenopausal women with hypoactive sexual desire." Fertil Steril 79(6): 1341-1352.
Long, C. Y., C. M. Liu, et al. (2006). "A randomized comparative study of the effects of oral and topical estrogen therapy on the vaginal vascularization and sexual function in hysterectomized postmenopausal women." Menopause 13(5): 737-743.
Myers, L. S., J. Dixen, et al. (1990). "Effects of estrogen, androgen, and progestin on sexual psychophysiology and behavior in postmenopausal women." J Clin Endocrinol Metab 70(4): 1124-1131.
Nathorst-Boos, J. and B. von Schoultz (1992). "Psychological reactions and sexual life after hysterectomy with and without oophorectomy." Gynecol Obstet Invest 34(2): 97-101.
Osborn, M., K. Hawton, et al. (1988). "Sexual dysfunction among middle aged women in the community." Br Med J (Clin Res Ed) 296(6627): 959-962.
Pelletier, G., J. Ouellet, et al. (2012a). "Effects of ovariectomy and dehydroepiandrosterone (DHEA) on vaginal innervation in the rat." J Sex Medicine: In press.
Pelletier, G., J. Ouellet, et al. (2012b). "Effects of Ovariectomy and Treatment with Prasterone, Estrogens and Acolbifene on Vaginal Innervation in The Rat." The North American Menopause Society (NAMS), " 23rd Annual Meeting: October 3-6, 2012, Orlando, Florida: S-18, 43-44.
Pessina, M. A., R. F. Hoyt, Jr., et al. (2006). "Differential effects of estradiol, progesterone, and testosterone on vaginal structural integrity." Endocrinology 147(1): 61-69. Rag
hunandan, C., S. Agrawal, et al. (2010). "A comparative study of the effects of local estrogen with or without local testosterone on vulvovaginal and sexual dysfunction in postmenopausal women." J Sex Med 7(3): 1284-1290.
Sarrel, P., B. Dobay, et al. (1998). "Estrogen and estrogen-androgen replacement in postmenopausal women dissatisfied with estrogen-only therapy. Sexual behavior and neuroendocrine responses." J Reprod Med 43(10): 847-856.
Sherwin, B. B. and M. M. Gelfand (1985). "Differential symptom response to parenteral estrogen and/or androgen administration in the surgical menopause." Am. J. Obstet. Gynecol. 151: 153-160.
Sherwin, B. B. and M. M. Gelfand (1987). "The role of androgen in the maintenance of sexual functioning in oophorectomized women." Psychosom Med. 49: 397-409.
Shifren, J. L., S. R. Davis, et al. (2006). "Testosterone patch for the treatment of hypoactive sexual desire disorder in naturally menopausal women: results from the INTIMATE NM1 Study." Menopause 13(5): 770-779.
Simon, J., E. Klaiber, et al. (1999). "Differential effects of estrogen-androgen and estrogen-only therapy on vasomotor symptoms, gonadotropin secretion, and endogenous androgen bioavailability in postmenopausal women." Menopause 6(2): 138-146. Tuit
en, A., J. Van Honk, et al. (2000). "Time course of effects of testosterone administration on sexual arousal in women." Arch Gen Psychiatry 57(2): 149-153; discussion 155-156.
Tuiten, A., J. van Honk, et al. (2002). "Can sublingual testosterone increase subjective and physiological measures of laboratory-induced sexual arousal?" Arch Gen Psychiatry 59(5): 465-466.
Villareal, D. T. and J. O. Holloszy (2004). "Effect of DHEA on abdominal fat and insulin action in elderly women and men: a randomized controlled trial." JAMA 292(18): 2243-2248.
Wiegel, M., C. Meston, et al. (2005). "The female sexual function index (FSFI): cross-validation and development of clinical cutoff scores." J Sex Marital Ther 31(1): 1-20.

**Table 1:**

| Average percentages of change from baseline to Week 12 of severity scores for sexual desire, avoiding intimacy, vaginal dryness, sexual domain as well as summary score of the four domains of the MENQOL questionnaire in all women (ITT population) as well as in the sub-groups of women without and with moderate/severe pain at sexual activity at baseline. | | | | | | | | | | | | | | | |
|---|---|---|---|---|---|---|---|---|---|---|---|---|---|---|---|
| | **All subjects (none, mild, moderate and severe pain at sexual activity)** | | | | | **Subjects with no moderate to severe (MS) pain at sexual activity** | | | | | **Subjects with moderate or severe (MS) pain at sexual activity** | | | | |
| **MENQOL** | **p values versus baseline** | **p values versus placebo** | | | | **p values versus baseline** | **p values versus placebo** | | | | **p values versus baseline** | **p values versus placebo** | | | |
| **Parameters - 12-week treatment** | **Placebo n=53** | **0.25% n=53** | **0.5% n=56** | **1.0% n=53-54** | **Pool 3 Doses n=163** | **Placebo n=12-13** | **0.25% n=15-16** | **0.5% n=13** | **1.0% n=14** | **Pool 3 Doses n=43-44** | **Placebo n=40** | **0.25% n=37** | **0.5% n=43** | **1.0% n=39** | **Pool 3 Doses n=119** |
| **Sexual desire (Q 27) (decreased from baseline)** | 22%* | 51%** | 31% | 48%** | 44.0%** | 6.1% | 61%** | +8.1% | 35% | 40.2%** | 27%* | 48% | 36% | 53%* | 45.0% |
| | 0.0116 | 0.0047 | 0.2845 | 0.0072 | 0.0083 | 0.4890 | 0.0002 | 0.3158 | 0.0551 | 0.0073 | 0.0154 | 0.1746 | 0.5876 | 0.0332 | 0.1058 |
| **Avoiding intimacy (Q 29) decreased from baseline)** | 22%** | 43%* | 41 %* | 50%** | 44.8%** | 9.2% | 49%* | 13%* | 27% | 36.6%* | 25%** | 41% | 43% | 56%** | 46.4%* |
| | 0.0072 | 0.0411 | 0.0431 | 0.0038 | 0.0047 | 0.6575 | 0.0233 | 0.05021 | 0.2715 | 0.0313 | 0.0051 | 0.2215 | 0.1463 | 0.0064 | 0.0271 |
| **Vaginal dryness during intercourse (Q28)(decreased from baseline)** | 23%** | 45%** | 50%** | 54%** | 49.9%** | 0% | 47%** | 31%* | 31%* | 38.2%** | 29%** | 45% | 52%** | 60%** | 52.4%** |
| | 0.0004 | 0.0032 | 0.0003 | <0.0001 | <00001 | 1.0000 | 0.0032 | 0.02341 | 003571 | 0.0028 | <0.0001 | 0.0700 | 0.0056 | 0.0004 | 0.0009 |
| **Sexual domain (decreased from baseline)** | 22%** | 47%** | 42%** | 51%** | 46.7%** | 4.3% | 53%** | 16%* | 31%* | 39.2%** | 27%** | 45%* | 45%* | 57%** | 48.4%** |
| | 0.0001 | 0.0003 | 0.0022 | <0.0001 | <0.0001 | 0.6944 | <0.0001 | 0.01941 | 0.0176 | 0.0003 | <0.0001 | 0.0298 | 0.0203 | 0.0001 | 0.0007 |
| **SUMMARY SCORE (decreased from baseline)** | 8.8%* | 24%** | 18% | 20%* | 20.8%** | 1.4% | 25%* | 12% | 14% | 18.0%* | 11%* | 24%** | 19% | 22%* | 21.4%* |
| | 0.0339 | 0.0006 | 0.0983 | 0.0135 | 0.0023 | 0.8413 | 0.0193 | 0.3278 | 0.1531 | 0.0475 | 0.0259 | 0.0080 | 0.1877 | 0.0371 | 0.0148 |

| | | | | | | | | | | | | | | | |
|---|---|---|---|---|---|---|---|---|---|---|---|---|---|---|---|
| * p<0.05; ** p<0.01 ¹: Significant versus Placebo but NS versus Baseline for this group LOCF was used for missing data | | | | | | | | | | | | | | | |

**Table 2:**

| Average percentages of change from baseline to Week 12 of severity scores for sexual desire, arousal/sensation, arousal/lubrication, orgasm and summary score of the Abbreviated Sexual Function (ASF) questionnaire in all women (ITT population) as well as in the sub-groups of women without and with moderate/severe pain at sexual activity at baseline. | | | | | | | | | | | | | | | |
|---|---|---|---|---|---|---|---|---|---|---|---|---|---|---|---|
| | **All subjects (none, mild, moderate and severe pain at sexual activity)** | | | | | **Subjects with no moderate to severe (MS) pain at sexual activity** | | | | | **Subjects with moderate or severe (MS) pain at sexual activity** | | | | |
| **ASF** | **p values versus baseline** | **p values versus placebo** | | | | **p values versus baseline** | **p values versus placebo** | | | | **p values versus baseline** | **p values versus placebo** | | | |
| **Parameters-12-week treatment** | **Placebo n=53** | **0.25% n=53** | **0.5% n=56** | **1.0% n=53-54** | **Pool 3 Doses n=163** | **Placebo n=12-13** | **0.25% n=15-16** | **0.5% n=13** | **1.0% n=14** | **Pool 3 Doses n=43-44** | **Placebo n=40** | **0.25% n=37** | **0.5% n=43** | **1.0% n=39** | **Pool 3 Doses n=119** |
| **Desire (increased from baseline)** | 8.7%* | 31%* | 13% | 22%* | 21.0%* | -1.2% | 31%* | 4.8% | 3.7% | 14.0% | 12%* | 32% | 16% | 29%* | 24.9% |
| | 0.0458 | 0.0260 | 0.5193 | 0.0322 | 0.0436 | 0.9028 | 0.0337 | 0.3706 | 0.5372 | 0.1273 | 0.0134 | 0.1349 | 0.8546 | 0.0341 | 0.1312 |
| **Arousal/sensa tion (increased from baseline)** | 8.6% | 80% | 72%* | 64%** | 71.2%** | -5.3% | 43% | 103% | 30% | 52.7% | 12% | 97% | 66% | 72%** | 76.2%* |
| | 0.5584 | 0.0673 | 0.0498 | 0.0039 | 0.0064 | 0.8912 | 0.3539 | 0.1137 | 0.4486 | 0.1963 | 0.4244 | 0.1024 | 0.1623 | 0.0023 | 0.0132 |
| **Arousal/ lubrication (increased from baseline)** | 45%* | 159%* | 159%** | 135%** | 149%** | 17% | 95% | 106% | 63% | 84.6% | 53%* | 189% | 170%* | 162%** | 171%** |
| | 0.0183 | 0.0233 | 0.0058 | 0.0007 | 0.0006 | 0.6465 | 0.1521 | 0.2200 | 0.2755 | 0.1254 | 0.0172 | 0.0556 | 0.0129 | 0.0007 | 0.0014 |
| **Orgasm (increased from baseline)** | 36%* | 64% | 53% | 71% | 62.4% | -4.1% | 59% | 56% | 50% | 55.3% | 50%* | 66% | 51% | 76% | 64.3% |
| | 0.0348 | 0.6808 | 0.4522 | 0.0838 | 0.2378 | 0.8816 | 0.2799 | 0.0737 | 0.3641 | 0.1297 | 0.0172 | 0.9851 | 0.9118 | 0.1000 | 0.5424 |
| **SUMMARY SCORE (increased from baseline)** | 16% | 54%* | 39% | 48%** | 46.6%** | -1.0% | 42% | 31% | 21% | 31.1% | 21%* | 59% | 42% | 57%** | 52.1%* |
| | 0.0563 | 0.0445 | 0.0984 | 0.0047 | 0.0083 | 0.9567 | 0.0881 | 0.1149 | 0.3073 | 0.0807 | 0.0226 | 0.1399 | 0.3085 | 0.0046 | 0.0306 |

| | | | | | | | | | | | | | | | |
|---|---|---|---|---|---|---|---|---|---|---|---|---|---|---|---|
| * p<0.05; ** p<0.01 LOCF was used for missing data | | | | | | | | | | | | | | | |

**Table 3:**

| Average percentages of change from baseline to Week 12 by DHEA treatment (average of the 3 doses) in the severity scores over placebo of the MENQOL and ASF questionnaires in all women (Total) as well as in the sub-groups of women without and with moderate or severe (MS) pain at baseline. | | | |
|---|---|---|---|
| **A- MENQOL** | **TOTAL** | **NO MS PAIN** | **MS PAIN** |
| **1-Sexual desire** | 22.0%** | 34.1%** | 18.0% |
| **2-Avoiding intimacy** | 22.8%** | 27.4%* | 21.4%* |
| **3-Vaginal dryness at sexual activity** | 26.9%** | 38.2%** | 23.4%** |
| **4-Sexual domain** | 24.7%** | 34.9%** | 21.4%** |
| **B- ASF** | **TOTAL** | **NO MS PAIN** | **MS PAIN** |
| **1-Desire** | 12.3%* | 15.2% | 12.9% |
| **2-Arousal/sensation** | 62.6%** | 58.0% | 64.2%* |
| **3-Arousal/lubrication** | 104%** | 67.6% | 118%** |
| **4-Orgasm** | 26.4% | 59.4% | 14.3% |
| **5-Summary score** | 30.6%** | 32.1% | 31.1%* |

**Table 4:**

| Average severity scores at baseline for sexual desire, avoiding intimacy, vaginal dryness as well as sexual domain summary of the MENQOL questionnaire in women without and with moderate/severe pain at sexual activity at baseline | | | | | | | | | | |
|---|---|---|---|---|---|---|---|---|---|---|
| | **Subjects with no moderate to severe (MS) pain at sexual activity** | | | | | **Subjects with moderate to severe (MS) pain at sexual activity** | | | | |
| **Prasterone concentration** | **0%** | **0.25%** | **0.5%** | **1.0%** | **Mean** | **0%** | **0.25%** | **0.5%** | **1.0%** | **Mean** |
| **Sexual desire** | 3.77 | 3.88 | 1.85 | 3.86 | 3.34 | 3.93 | 5.00 | 4.30 | 4.28 | 4.38 |
| **(maximum score=8)** | (13) | (16) | (13) | (14) | (56) | (40) | (37) | (43) | (39) | (159) |
| **Avoiding intimacy** | 3.58 | 3.69 | 1.15 | 3.43 | 2.96 | 4.45 | 5.16 | 5.05 | 4.46 | 4.78 |
| **(maximum score=8)** | (12) | (16) | (13) | (14) | (55) | (40) | (37) | (43) | (39) | (159) |
| **Vaginal dryness during sexual activity** | 5.15 | 4.87 | 3.23 | 4.43 | 4.42 | 6.68 | 6.84 | 6.84 | 6.67 | 6.76 |
| **(maximum score=8)** | (13) | (15) | (13) | (14) | (55) | (40) | (37) | (43) | (39) | (159) |
| **Sexual domain** | 4.19 | 4.21 | 2.08 | 3.90 | 3.60 | 5.02 | 5.67 | 5.40 | 5.14 | 5.31 |
| **(maximum score=8)** | (13) | (16) | (13) | (14) | (56) | (40) | (37) | (43) | (39) | (159) |

**Table 5:**

| Average severity scores at baseline for sexual desire, arousal/sensation, arousal/lubrication, orgasm and summary score of the Abbreviated Sexual Function (ASF) questionnaire in women without and with moderate/severe pain at sexual activity at baseline | | | | | | | | | | |
|---|---|---|---|---|---|---|---|---|---|---|
| | **Subjects with no moderate to severe (MS) pain at sexual activity** | | | | | **Subjects with moderate to severe (MS) pain at sexual activity** | | | | |
| **Prasterone concentration** | **0%** | **0.25%** | **0.5%** | **1.0%** | **Mean** | **0%** | **0.25%** | **0.5%** | **1.0%** | **Mean** |
| **Sexual desire** | 12.46 | 10.63 | 14.31 | 13.36 | 12.69 | 12.48 | 11.14 | 12.00 | 12.44 | 12.02 |
| **(maximum=30)** | (13) | (16) | (13) | (14) | | (40) | (37) | (43) | (39) | |
| **Arousal/sensation** | 4.38 | 3.19 | 2.54 | 3.53 | 3.41 | 5.05 | 3.22 | 3.81 | 5.03 | 4.28 |
| **(maximum=20)** | (13) | (16) | (13) | (15) | | (40) | (37) | (43) | (39) | |
| **Arousal/lubrication** | 1.85 | 1.38 | 1.08 | 1.80 | 1.53 | 1.98 | 1.22 | 1.49 | 1.87 | 1.64 |
| **(maximum=10)** | (13) | (16) | (13) | (15) | | (40) | (37) | (43) | (39) | |
| **Orgasm** | 3.69 | 2.63 | 4.15 | 2.67 | 3.29 | 3.53 | 2.97 | 3.35 | 4.00 | 3.46 |
| **(maximum=15)** | (13) | (16) | (13) | (15) | | (40) | (37) | (43) | (39) | |
| **Summary score** | 22.38 | 17.81 | 22.08 | 20.47 | 20.69 | 23.03 | 18.54 | 20.65 | 23.33 | 21.39 |
| | (13) | (16) | (13) | (15) | | (40) | (37) | (43) | (39) | |

### BRIEF DESCRIPTION OF THE DRAWINGS

**Figure 1****:** Effect of daily intravaginal application of 0.0%, 0.25%, 0.5% and 1.0% dehydroepiandrosterone (DHEA; Prasterone) for 12 weeks on the score of sexual desire of the Menopause-Specific Quality of Life (MENQOL) questionnaire in postmenopausal women without or with moderate or severe pain at sexual activity at baseline. Data are also presented for the sum of the three prasterone doses. Data are expressed as means ± SEM; the p values are comparisons with placebo at all DHEA doses except for the placebo group (0% DHEA) which is compared to baseline.
**Figure 2****:** Effect of daily intravaginal application of 0.0%, 0.25%, 0.5% and 1.0% dehydroepiandrosterone (DHEA; Prasterone) for 12 weeks on the score of avoiding intimacy of the Menopause-Specific Quality of Life (MENQOL) questionnaire in postmenopausal women without or with moderate or severe pain at sexual activity at baseline. Data are also presented for the sum of the three prasterone doses. Data are expressed as means ± SEM; the p values are comparisons with placebo at all DHEA doses except for the placebo group (0%DHEA) which is compared to baseline.
**Figure 3****:** Effect of daily intravaginal application of 0.0%, 0.25%, 0.5% and 1.0% dehydroepiandrosterone (DHEA; Prasterone) for 12 weeks on the score of vaginal dryness during intercourse of the Menopause-Specific Quality of Life (MENQOL) questionnaire in postmenopausal women without or with moderate or severe pain at sexual activity at baseline. Data are also presented for the sum of the three prasterone doses. Data are expressed as means ± SEM; the p values are comparisons with placebo at all DHEA doses except for the placebo group (0% DHEA) which is compared to baseline.
**Figure 4****:** Effect of daily intravaginal application of 0.0%, 0.25%, 0.5% and 1.0% dehydroepiandrosterone (DHEA; Prasterone) for 12 weeks on the summary score of the Menopause-Specific Quality of Life (MENQOL) questionnaire in postmenopausal women without or with moderate or severe pain at sexual activity at baseline. Data are also presented for the sum of the three prasterone doses. Data are expressed as means ± SEM; the p values are comparisons with placebo at all DHEA doses except for the placebo group (0% DHEA) which is compared to baseline.
**Figure 5****:** Effect of daily intravaginal application of 0.0%, 0.25%, 0.5% and 1.0% dehydroepiandrosterone (DHEA; Prasterone) for 12 weeks on the score of desire domain of the Abbreviated Sexual Function (ASF) questionnaire in postmenopausal women without or with moderate or severe pain at sexual activity at baseline. Data are also presented for the sum of the three prasterone doses. Data are expressed as means ± SEM; the p values are comparisons with placebo at all DHEA doses except for the placebo group (0% DHEA) which is compared to baseline.
**Figure 6****:** Effect of daily intravaginal application of 0.0%, 0.25%, 0.5% and 1.0% dehydroepiandrosterone (DHEA; Prasterone) for 12 weeks on the score of the arousal-sensation domain of the Abbreviated Sexual Function (ASF) questionnaire in postmenopausal women without or with moderate or severe pain at sexual activity at baseline. Data are also presented for the sum of the three prasterone doses. Data are expressed as means ± SEM; the p values are comparisons with placebo at all DHEA doses except for the placebo group (0% DHEA) which is compared to baseline.
**Figure 7****:** Effect of daily intravaginal application of 0.0%, 0.25%, 0.5% and 1.0% dehydroepiandrosterone (DHEA; Prasterone) for 12 weeks on the score of the arousal-lubrication domain of the Abbreviated Sexual Function (ASF) questionnaire in postmenopausal women without or with moderate or severe pain at sexual activity at baseline. Data are also presented for the sum of the three prasterone doses. Data are expressed as means ± SEM; the p values are comparisons with placebo at all DHEA doses except for the placebo group (0% DHEA) which is compared to baseline.
**Figure 8****:** Effect of daily intravaginal application of 0.0%, 0.25%, 0.5% and 1.0% dehydroepiandrosterone (DHEA; Prasterone) for 12 weeks on the score of the orgasm domain of the Abbreviated Sexual Function (ASF) questionnaire in postmenopausal women without or with moderate or severe pain at sexual activity at baseline. Data are also presented for the sum of the three prasterone doses. Data are expressed as means ± SEM; the p values are comparisons with placebo at all DHEA doses except for the placebo group (0% DHEA) which is compared to baseline.
**Figure 9****:** Average changes over placebo of the severity scores of the MENQOL and ASF questions/domains induced by the daily intravaginal administration of 0.25%, 0.5% and 1.0% DHEA in the total groups (all women) and in women without and with moderate or severe pain at sexual activity at baseline.
**Figure 10****:** Comparison of the effect of daily intravaginal application of a placebo suppository for 12 weeks on the placebo effects expressed as % of baseline in response to the sexual domain of the MENQOL and the domains of the Abbreviated Sexual Function (ASF) questionnaires in postmenopausal women without (non MS) and with (MS) moderate/severe pain at sexual activity at baseline.
**Figure 11****:** Comparison of the placebo effects and changes over placebo in the non MS and MS groups at baseline (total of all women with and without MS pain at baseline are included for comparison) for the various questions/domains of the MENQOL (A) and ASF (B) questionnaires. Data are from Tables 1, 2 and 3; *, p<0.05 over placebo and ** p<0.01 over placebo (prasterone effect).

## Claims

1. Intravaginal DHEA for use in a method for the treatment of at least one condition selected from the group consisting of female hypoactive sexual desire disorder, female sexual arousal disorder, female orgasm disorder and female sexual interest arousal disorder in a woman who is not suffering from symptoms of vulvo-vaginal atrophy; wherein the symptoms of vulvo-vaginal atrophy are selected from the group consisting of vaginal dryness, burning and itching, and dyspareunia.

2. Pharmaceutical composition comprising intravaginal DHEA for use in a method for the treatment of at least one condition selected from the group consisting of female hypoactive sexual desire disorder, female sexual arousal disorder, female orgasm disorder and female sexual interest arousal disorder in a woman who is not suffering from symptoms of vulvo-vaginal atrophy; wherein the symptoms of vulvo-vaginal atrophy are selected from the group consisting of vaginal dryness, burning and itching, and dyspareunia.

3. The intravaginal DHEA of claim 1 wherein the woman is pre-menopausal.

4. The intravaginal DHEA of claim 1 wherein the woman is post-menopausal.

5. The pharmaceutical composition of claim 2 wherein the woman is pre-menopausal.

6. The pharmaceutical composition of claim 2 wherein the woman is post-menopausal.

## Patentansprüche

1. Intravaginales DHEA (Dehydroepiandrosteron) zur Verwendung bei einer Methode zur Behandlung von mindestens einem Zustand, der aus der Gruppe ausgewählt ist, die aus gestörtem minderaktivem sexuellem Verlangen der Frau, gestörter sexueller Erregung der Frau, gestörtem weiblichem Orgasmus und gestörtem Interesse der Frau an sexueller Erregung bei einer Frau besteht, die nicht an Symptomen einer vulva-vaginalen Atrophie leidet; wobei die Symptome von vulva-vaginaler Atrophie aus der Gruppe ausgewählt sind, die aus Scheidentrockenheit, Brennen und Juckreiz sowie Orgasmusunfähigkeit besteht.

2. Pharmazeutische Zusammensetzung, enthaltend intravaginales DHEA zur Verwendung bei einer Methode zur Behandlung von mindestens einem Zustand, der aus der Gruppe ausgewählt ist, die aus gestörtem minderaktivem sexuellem Verlangen der Frau, gestörter sexueller Erregung der Frau, gestörtem weiblichem Orgasmus und gestörtem Interesse der Frau an sexueller Erregung bei einer Frau besteht, die nicht an Symptomen einer vulva-vaginalen Atrophie leidet; wobei die Symptome von vulva-vaginaler Atrophie aus der Gruppe ausgewählt sind, die aus Scheidentrockenheit, Brennen und Juckreiz sowie Orgasmusunfähigkeit besteht.

3. Intravaginales DHEA nach Anspruch 1, wobei sich die Frau vor der Menopause befindet.

4. Intravaginales DHEA nach Anspruch 1, wobei sich die Frau nach der Menopause befindet.

5. Pharmazeutische Zusammensetzung nach Anspruch 2, wobei sich die Frau vor der Menopause befindet.

6. Pharmazeutische Zusammensetzung nach Anspruch 2, wobei sich die Frau nach der Menopause befindet.

## Revendications

1. DHA (déhydroépiandrostérone) intra-vaginale pour utilisation dans un procédé pour le traitement d'au moins un état sélectionné à partir du groupe constitué d'un trouble du désir sexuel hypoactif féminin, d'un trouble de l'excitation sexuelle féminine, d'un trouble de l'orgasme féminin et d'un trouble de l'intérêt / excitation sexuel féminin chez une femme qui ne souffre pas de symptômes d'atrophie vulvo-vaginale ; dans laquelle les symptômes de l'atrophie vulvo-vaginale sont sélectionnés à partir du groupe constitué de la sécheresse vaginale, de brûlures vaginales et de prurit vaginal, et de la dyspareunie.

2. Composition pharmaceutique comprenant de la DHA intra-vaginale pour utilisation dans un procédé pour le traitement d'au moins un état sélectionné à partir du groupe constitué d'un trouble du désir sexuel hypoactif féminin, d'un trouble de l'excitation sexuelle féminine, d'un trouble de l'orgasme féminin et d'un trouble de l'intérêt / excitation sexuel féminin chez une femme qui ne souffre pas de symptômes d'atrophie vulvo-vaginale ; dans laquelle les symptômes de l'atrophie vulvo-vaginale sont sélectionnés à partir du groupe constitué de la sécheresse vaginale, de brûlures vaginales et de prurit vaginal, et de la dyspareunie.

3. DHA intra-vaginale selon la revendication 1 dans laquelle la femme est en pré-ménopause.

4. DHA intra-vaginale selon la revendication 1 dans laquelle la femme est en post-ménopause.

5. Composition pharmaceutique selon la revendication 2 dans laquelle la femme est en pré-ménopause.

6. Composition pharmaceutique selon la revendication 2 dans laquelle la femme est en post-ménopause.
